# EUROPEAN PATENT APPLICATION

(11) **EP 4 516 898 A1**
(43) Date of publication of application: **05.03.2025**
(21) Application number: 23796468.9
(22) Date of filing: 27.04.2023
(51) Int. Cl.: C12N 7/01, A61K 35/76, A61P 11/00, A61P 31/04, C12N 7/00, C12N 15/33

(54) **BACTERIOPHAGE**

(30) Priority: 28.04.2022 JP 2022075078
(71) Applicant: Astellas Pharma, Inc., Chuo-ku Tokyo 103-8411 (JP); NATIONAL UNIVERSITY CORPORATION TOKAI NATIONAL HIGHER EDUCATION AND RESEARCH SYSTEM, Nagoya-shi, Aichi 464-8601 (JP)
(72) Inventor: ANDO Hiroki, Tokyo 103-8411 (JP); MATSUOKA Hideaki, Tokyo 103-8411 (JP); MATSUKAWA Koji, Tokyo 103-8411 (JP); ONO Yutaro, Tokyo 103-8411 (JP); MITSUNAKA Shoichi, Gifu-shi, Gifu 501-1193 (JP)
(74) Representative: Hoffmann Eitle
(86) International application number: PCT/JP2023/016600
(87) International publication number: WO 2023/210732

(57) **Abstract**

It is an object of the present invention to provide a bacteriophage having lytic activity against *Mycobacterium avium* and/or *Mycobacterium intracellulare.* The present invention provides a bacteriophage having lytic activity against *Mycobacterium avium* and/or *Mycobacterium intracellulare,* wherein the genome of the bacteriophage contains the nucleic acid sequence of the genome of a bacteriophage identified by any of Accession Nos. NITE BP-03513 to NITE BP-03521.

## Description

### Field of the Invention

The present invention relates to bacteriophages and uses of the bacteriophages.

### Background Art

Non-tuberculous mycobacteria (NTM) disease is an infection by acid-fast bacteria other than *Mycobacterium tuberculosis* and *Mycobacterium leprae.* NTM is environment resident bacteria that exist in lakes, marshes, soil, and the like, and causes respiratory symptoms when inhaled as an aerosol or the like. Examples of NTM include *Mycobacterium avium* (*M. avium*), *Mycobacterium intracellulare* (*M. intracellulare*)*, Mycobacterium fortuitum* (*M. fortuitum*)*, Mycobacterium chelonae* (*M. chelonae*)*, Mycobacterium gordonae* (*M. gordonae*), *Mycobacterium szulagai* (*M. szulgai*)*, Mycobacterium kansasii* (*M. kansasii*), and *Mycobacterium genavense* (*M. genavense*) (Infection. 2004; 32: 257.). Among them, infections caused by *Mycobacterium avium* complex (MAC) including *Mycobacterium avium* and *Mycobacterium intracellulare* are called MAC disease, of which pulmonary infections are particularly called pulmonary MAC disease.

Therapeutic methods for bacterial infections including NTM disease are generally multidrug therapy containing macrolide antibiotics, but in recent years, phage therapy using the lytic activity of bacteriophages (hereinafter also referred to as phages) have been known (Microorganisms. 2021; 9 (3): 596.). For example, D29, TM4, and ZoeJ have been reported as phages having lytic activity against *Mycobacterium avium* (Journal of Medical Microbiology 2006;55(1):37.; Microorganisms. 2021; 9 (3): 596.; Microb. Drug Resist. 2006; 12: 1.).

A bacteriophage is a virus whose host is a bacterium. A phage that infects a specific bacterium injects its own genome into the bacterium and uses the metabolic mechanism of the host bacterium to propagate its progeny phage. Thereafter, the bacterial cell wall is destructed by the lytic enzymes carried in the phage genome, the progeny phage are released, and the host bacterium is killed.

Phages can be broadly classified into non-temperate phages (lytic phages) and temperate phages (lysogenic phages) (Scientifica (Cairo). 2014; 2014: 581639.). Non-temperate phages replicate themselves without integrating into the host bacterial DNA. Progeny phages are propagated within the bacterium, resulting in lysis of the bacterium and release of mature phage particles. Meanwhile, after infection with bacteria, temperate phages integrate into the host bacterial DNA and replicate themselves together with the host bacterial DNA. It is harmless to the host bacterium just by integrating into the host bacterial DNA, and it is possible to maintain a lysogenized state (a state in which the phage DNA integrates into the host bacterial DNA and replicates itself with the host bacterial DNA). However, when temperate phages are exposed to an external stimulus such as ultraviolet rays, the temperate phages release a lytic enzyme like non-temperate phages, lyse the host bacterium, and release mature phage particles.

Phages lacking genes involved in elements of lysogenization have also been reported. For example, a phage lacking integrase gene (Nat Med. 2019; 25 (5): 730.) and a phage lacking integrase gene and immunity repressor gene (mBio. 2021; 12 (3): e00973.) have been reported.

### Technical Problem[0007]

An object of the present invention is to provide a bacteriophage having lytic activity against *Mycobacterium avium* and/or *Mycobacterium intracellulare.* It is another object of the present invention to provide a means and a method, particularly a bacteriophage and a pharmaceutical composition, for preventing or treating nontuberculous mycobacterial disease such as pulmonary MAC disease.

### Solution to Problem

As a result of creative studies in producing bacteriophages, the present inventors have obtained new bacteriophages and gene-deficient strain lacking their lysogenic genes (Example 1) and have found that the bacteriophages have lytic activity against *Mycobacterium avium* and *Mycobacterium intracellulare* (Example 2), thereby accomplishing the present invention. Further, the present inventors have found that a pharmaceutical composition containing the bacteriophages identified by Accession No. NITE BP-03513, Accession No. NITE BP-03514, and Accession No. NITE BP-03519 has excellent bactericidal activity as compared with individual bacteriophages (Example 3).

Specifically, the present invention may include the following inventions as materials or methods that are medically or industrially useful.
[1] A bacteriophage selected from (1) to (9) below:
   (1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks an integrase gene and/or an immunity repressor gene;
   (2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks an integrase gene and/or an immunity repressor gene;
   (3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03515; and (b) lacks an integrase gene and/or an immunity repressor gene;
   (4) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03516; and (b) lacks an integrase gene and/or an immunity repressor gene;
   (5) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03517; and (b) lacks an integrase gene and/or an immunity repressor gene;
   (6) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03518;
   (7) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519;
   (8) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03520; and
   (9) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03521.
[2] The bacteriophage according to [1], which is selected from (1) to (9) below:
   (1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
   (2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514;
   (3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03515;
   (4) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03516;
   (5) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03517;
   (6) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03518;
   (7) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519;
   (8) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03520; and
   (9) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03521.
[3] The bacteriophage according to [1] or [2], which is selected from (1) to (9) below:
   (1) a bacteriophage wherein a genome of the bacteriophage consists of a nucleic acid sequence of a genome of the bacteriophage identified by Accession No. NITE BP-03513;
   (2) a bacteriophage wherein a genome of the bacteriophage consists of a nucleic acid sequence of a genome of the bacteriophage identified by Accession No. NITE BP-03514;
   (3) a bacteriophage wherein a genome of the bacteriophage consists of a nucleic acid sequence of a genome of the bacteriophage identified by Accession No. NITE BP-03515;
   (4) a bacteriophage wherein a genome of the bacteriophage consists of a nucleic acid sequence of a genome of the bacteriophage identified by Accession No. NITE BP-03516;
   (5) a bacteriophage wherein a genome of the bacteriophage consists of a nucleic acid sequence of a genome of the bacteriophage identified by Accession No. NITE BP-03517;
   (6) a bacteriophage wherein a genome of the bacteriophage consists of a nucleic acid sequence of a genome of the bacteriophage identified by Accession No. NITE BP-03518;
   (7) a bacteriophage wherein a genome of the bacteriophage consists of a nucleic acid sequence of a genome of the bacteriophage identified by Accession No. NITE BP-03519;
   (8) a bacteriophage wherein a genome of the bacteriophage consists of a nucleic acid sequence of a genome of the bacteriophage identified by Accession No. NITE BP-03520; and
   (9) a bacteriophage wherein a genome of the bacteriophage consists of a nucleic acid sequence of a genome of the bacteriophage identified by Accession No. NITE BP-03521.
[4] A bacteriophage selected from (1) to (9) below:
   (1) the bacteriophage identified by Accession No. NITE BP-03513 or a passage strain thereof;
   (2) the bacteriophage identified by Accession No. NITE BP-03514 or a passage strain thereof;
   (3) the bacteriophage identified by Accession No. NITE BP-03515 or a passage strain thereof;
   (4) the bacteriophage identified by Accession No. NITE BP-03516 or a passage strain thereof;
   (5) the bacteriophage identified by Accession No. NITE BP-03517 or a passage strain thereof;
   (6) the bacteriophage identified by Accession No. NITE BP-03518 or a passage strain thereof;
   (7) the bacteriophage identified by Accession No. NITE BP-03519 or a passage strain thereof;
   (8) the bacteriophage identified by Accession No. NITE BP-03520 or a passage strain thereof; and
   (9) the bacteriophage identified by Accession No. NITE BP-03521 or a passage strain thereof.
[5] A pharmaceutical composition comprising the bacteriophage according to any one of [1] to [4] and a pharmaceutically acceptable excipient.
[6] A pharmaceutical composition comprising at least two bacteriophages according to any one of [1] to [4], and a pharmaceutically acceptable excipient.
[7] A pharmaceutical composition comprising three bacteriophages according to any one of [1] to [4] and a pharmaceutically acceptable excipient.
[8] The pharmaceutical composition according to any one of [5] to [7], which is a pharmaceutical composition for preventing or treating nontuberculous mycobacterial disease.
[9] The pharmaceutical composition according to [8], wherein the nontuberculous mycobacterial disease is pulmonary MAC disease.
[10] A pharmaceutical composition comprising the bacteriophages of the following (1) to (3) and a pharmaceutically acceptable excipient:
   (1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks an integrase gene and/or an immunity repressor gene;
   (2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks an integrase gene and/or an immunity repressor gene; and
   (3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.
[11] The pharmaceutical composition according to [10], comprising the bacteriophages of the following (1) to (3):
   (1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
   (2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514; and
   (3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519.
[12] A pharmaceutical composition according to [10] or [11], comprising the bacteriophages of the following (1) to (3):
   (1) a bacteriophage wherein a genome of the bacteriophage consists of a nucleic acid sequence of a genome of the bacteriophage identified by Accession No. NITE BP-03513;
   (2) a bacteriophage wherein a genome of the bacteriophage consists of a nucleic acid sequence of a genome of the bacteriophage identified by Accession No. NITE BP-03514; and
   (3) a bacteriophage wherein a genome of the bacteriophage consists of a nucleic acid sequence of a genome of the bacteriophage identified by Accession No. NITE BP-03519.
[13] A pharmaceutical composition comprising the bacteriophages of the following (1) to (3) and a pharmaceutically acceptable excipient:
   (1) the bacteriophage identified by Accession No. NITE BP-03513 or a passage strain thereof;
   (2) the bacteriophage identified by Accession No. NITE BP-03514 or a passage strain thereof; and
   (3) the bacteriophage identified by Accession No. NITE BP-03519 or a passage strain thereof.
[14] The pharmaceutical composition according to any one of [10] to [13], which is a pharmaceutical composition for preventing or treating nontuberculous mycobacterial disease.
[15] The pharmaceutical composition according to [14], wherein the nontuberculous mycobacterial disease is pulmonary MAC disease.

### Advantageous Effects of Invention

The bacteriophage and the pharmaceutical composition of the present invention have lytic activity against *Mycobacterium avium* and/or *Mycobacterium intracellulare,* and the bacteriophage and the pharmaceutical composition of the present invention are expected to be useful for preventing or treating nontuberculous mycobacterial disease such as pulmonary MAC disease.

### BRIEF DESCRIPTION OF THE DRAWINGS

[FIG. 1] FIG. 1 shows a specific example of lytic plaques. By visual inspection, lytic plaques with clear contours and little or no turbidity were determined as +++, lytic plaques with clear contours and turbidity were determined as ++, and lytic plaques with unclear contours and turbidity were determined as +.
[FIG. 2] FIG. 2 is a graph showing the results of the lytic activity of each bacteriophage over time. The vertical axis indicates colony forming unit concentration (CFU/mL), and the horizontal axis indicates time after initiation of culture. LOQ represents the lower limit of quantification.
[FIG. 3] FIG. 3 a graph showing the results of bactericidal activity of the bacteriophages in a mouse infection model. The vertical axis indicates colony forming units in lungs (CFU/lung). LOQ represents the lower limit of quantification.

### DETAILED DESCRIPTION OF EMBODIMENTS

Hereinafter, the present invention will be described in detail. The following embodiments are examples for explaining the present invention and are not intended to limit the present invention only to these embodiments. The present invention can be embodied in various embodiments without departing from the gist thereof.

### < 1. Bacteriophage of the present invention >

In one embodiment, the present invention provides a new bacteriophage. The bacteriophage according to the present invention is a bacteriophage having lytic activity against non-tuberculous mycobacteria (NTM), particularly *Mycobacterium avium* and/or *Mycobacterium intracellulare,* which cause pulmonary MAC disease (which may be hereinafter referred to as "bacteriophage of the present invention").

The D29 lysogenic gene-deficient strain D29Δ, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by one of the Applicants on August 26, 2021 (Accession No. NITE BP-03513).

The B1 lysogenic gene-deficient strain B1Δ, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03514).

The Y2 lysogenic gene-deficient strain Y2Δ, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03515).

The #33 lysogenic gene-deficient strain #33Δ, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03516).

The #63 lysogenic gene-deficient strain #63Δ, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03517).

The #105 parent strain, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03518).

The #123 parent strain, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03519).

The #33 parent strain, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03520).

The #63 parent strain, which is an example of the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) by one of the Applicants on August 26, 2021 (Accession No. NITE BP-03521).

In one embodiment, the bacteriophage of the present invention may include a bacteriophage(s) selected from the group consisting of (1) to (9) below:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks a lysogenic gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks a lysogenic gene;
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03515; and (b) lacks a lysogenic gene;
(4) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03516; and (b) lacks a lysogenic gene;
(5) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03517; and (b) lacks a lysogenic gene;
(6) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03518;
(7) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519;
(8) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03520; and
(9) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03521.

The "identity" used herein means the value Identity obtained using parameters prepared by default by NEEDLE program (J. Mol. Biol., 1970, Vol. 48, p. 443.) search. The parameters are as follows.
Gap penalty = 10
Extend penalty = 0.5
Matrix = EBLOSUM62

The "lysogenic gene(s)" used herein means a gene(s) involved in the induction and/or maintenance of a lysogenic state. Examples of the "lysogenic gene(s)" may include integrase genes, immunity repressor genes, and Cro genes. Specifically, lysogenic genes and their sequence information are also known (Nat Med. 2019; 25 (5): 730.; mBio. 2021; 12 (3): e00973.; Cell. 2018; 172 (6): 1260.).

As used herein, "lacking a lysogenic gene" means that 1) the full-length of at least one lysogenic gene is lacking from the sequence of the genome of the bacteriophage, or 2-1) the bacteriophage becomes a lytic bacteriophage by modifying a part of at least one lysogenic gene region by deletion, substitution, insertion, addition, or a combination thereof from the sequence of the genome of the bacteriophage, or 2-2) the function of the lysogenic gene is lacking. The lysogenic gene can be deleted by a known method in the art depending on the type of the lysogenic gene to be deleted (e.g., Nat Med. 2019; 25 (5): 730.; mBio. 2021; 12 (3): e00973.; PLoS One. 2008; 3 (12): e3957.).

As used herein, "having lytic activity against *Mycobacterium avium* and/or *Mycobacterium intracellulare"* means to show lytic plaques against at least one strain of bacteria belonging to *Mycobacterium avium* or *Mycobacterium intracellulare.* Confirmation of lytic activity against these bacteria can be performed using a method and device known in the art. For example, whether or not a bacteriophage has lytic activity can be confirmed using the method according to Example 2 in this description by confirming whether or not 10⁹ pfu/mL bacteriophage exhibits lytic plaques against *Mycobacterium avium* or *Mycobacterium intracellulare.*

In one embodiment, the bacteriophage of the present invention may include a bacteriophage selected from the group consisting of (1) to (9) below:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks an integrase gene and/or an immunity repressor gene;
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03515; and (b) lacks an integrase gene and/or an immunity repressor gene;
(4) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03516; and (b) lacks an integrase gene and/or an immunity repressor gene;
(5) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03517; and (b) lacks an integrase gene and/or an immunity repressor gene;
(6) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03518;
(7) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519;
(8) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03520; and
(9) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03521.

The phrase "lacking an integrase gene and/or an immunity repressor gene" means 1) the full-length of the integrase gene and/or the immunity repressor gene is deleted from the sequence of the genome of the bacteriophage, or 2-1) the bacteriophage becomes a lytic bacteriophage by modifying a part of the integrase gene and/or the immunity repressor gene from the sequence of the genome of the bacteriophage by deletion, substitution, insertion, addition, or a combination thereof, or 2-2) the function of the integrase gene and/or the immunity repressor gene is deficient. As mentioned above, a lysogenic gene such as an integrase gene and an immunity repressor gene can be deleted by a method known in the art depending on the type of the lysogenic gene to be deleted (e.g., Nat Med. 2019; 25 (5): 730.; mBio. 2021; 12 (3): e00973.; PLoS One. 2008; 3 (12): e3957.).

In one embodiment, the bacteriophage of the present invention may include a bacteriophage having lytic activity against *Mycobacterium avium* and/or *Mycobacterium intracellulare,* wherein a genome of the bacteriophage: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03513; and (b) lacks an integrase gene and/or an immunity repressor gene.

In one embodiment, the bacteriophage of the present invention may include a bacteriophage having lytic activity against *Mycobacterium avium* and/or *Mycobacterium intracellulare,* wherein a genome of the bacteriophage: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03514; and (b) lacks an integrase gene and/or an immunity repressor gene.

In one embodiment, the bacteriophage of the present invention may include a bacteriophage having lytic activity against *Mycobacterium avium* and/or *Mycobacterium intracellulare,* wherein a genome of the bacteriophage: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03515; and (b) lacks an integrase gene and/or an immunity repressor gene.

In one embodiment, the bacteriophage of the present invention may include a bacteriophage having lytic activity against *Mycobacterium avium* and/or *Mycobacterium intracellulare,* wherein a genome of the bacteriophage: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03516; and (b) lacks an integrase gene and/or an immunity repressor gene.

In one embodiment, the bacteriophage of the present invention may include a bacteriophage having lytic activity against *Mycobacterium avium* and/or *Mycobacterium intracellulare,* wherein a genome of the bacteriophage: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03517; and (b) lacks an integrase gene and/or an immunity repressor gene.

In one embodiment, the bacteriophage of the present invention may include a bacteriophage having lytic activity against *Mycobacterium avium* and/or *Mycobacterium intracellulare,* wherein a genome of the bacteriophage comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03518.

In one embodiment, the bacteriophage of the present invention my include a bacteriophage having lytic activity against *Mycobacterium avium* and/or *Mycobacterium intracellulare,* wherein a genome of the bacteriophage comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03519.

In one embodiment, the bacteriophage of the present invention may include a bacteriophage having lytic activity against *Mycobacterium avium* and/or *Mycobacterium intracellulare,* wherein a genome of the bacteriophage comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03520.

In one embodiment, the bacteriophage of the present invention may include a bacteriophage having lytic activity against *Mycobacterium avium* and/or *Mycobacterium intracellulare,* wherein a genome of the bacteriophage comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03521.

The bacteriophage of the present invention may include a bacteriophage selected from (1) to (9) below:
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514;
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03515;
(4) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03516;
(5) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03517;
(6) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03518;
(7) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519;
(8) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03520; and
(9) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03521.

The bacteriophage of the present invention may include a bacteriophage selected from (1) to (9) below:
(1) a bacteriophage wherein a genome of the bacteriophage consists of a nucleic acid sequence of a genome of the bacteriophage identified by Accession No. NITE BP-03513;
(2) a bacteriophage wherein a genome of the bacteriophage consists of a nucleic acid sequence of a genome of the bacteriophage identified by Accession No. NITE BP-03514;
(3) a bacteriophage wherein a genome of the bacteriophage consists of a nucleic acid sequence of a genome of the bacteriophage identified by Accession No. NITE BP-03515;
(4) a bacteriophage wherein a genome of the bacteriophage consists of a nucleic acid sequence of a genome of the bacteriophage identified by Accession No. NITE BP-03516;
(5) a bacteriophage wherein a genome of the bacteriophage consists of a nucleic acid sequence of a genome of the bacteriophage identified by Accession No. NITE BP-03517;
(6) a bacteriophage wherein a genome of the bacteriophage consists of a nucleic acid sequence of a genome of the bacteriophage identified by Accession No. NITE BP-03518;
(7) a bacteriophage wherein a genome of the bacteriophage consists of a nucleic acid sequence of a genome of the bacteriophage identified by Accession No. NITE BP-03519;
(8) a bacteriophage wherein a genome of the bacteriophage consists of a nucleic acid sequence of a genome of the bacteriophage identified by Accession No. NITE BP-03520; and
(9) a bacteriophage wherein a genome of the bacteriophage consists of a nucleic acid sequence of a genome of the bacteriophage identified by Accession No. NITE BP-03521.

The bacteriophage of the present invention may also include a bacteriophage selected from (1) to (9) below:
(1) the bacteriophage identified by Accession No. NITE BP-03513;
(2) the bacteriophage identified by Accession No. NITE BP-03514;
(3) the bacteriophage identified by Accession No. NITE BP-03515;
(4) the bacteriophage identified by Accession No. NITE BP-03516;
(5) the bacteriophage identified by Accession No. NITE BP-03517;
(6) the bacteriophage identified by Accession No. NITE BP-03518;
(7) the bacteriophage identified by Accession No. NITE BP-03519;
(8) the bacteriophage identified by Accession No. NITE BP-03520; and
(9) the bacteriophage identified by Accession No. NITE BP-03521.

The bacteriophage of the present invention may also include a bacteriophage selected from (1) to (9) below:
(1) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03513 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03514 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene;
(3) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03515 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene;
(4) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03516 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene;
(5) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03517 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene;
(6) a bacteriophage having lytic activity against *Mycobacterium avium* and/or *Mycobacterium intracellulare,* wherein a genome of the bacteriophage comprises a nucleic acid sequence modified from a nucleic acid sequence of a genome of the bacteriophage identified by Accession No. NITE BP-03518 by deletion, substitution, insertion, or addition of 1 to 5000 (e.g., 1 to 1000, 1 to 500, or 1 to 100) nucleotides, or a combination thereof;
(7) a bacteriophage having lytic activity against *Mycobacterium avium* and/or *Mycobacterium intracellulare,* wherein a genome of the bacteriophage comprises a nucleic acid sequence modified from a nucleic acid sequence of a genome of the bacteriophage identified by Accession No. NITE BP-03519 by deletion, substitution, insertion, or addition of 1 to 5000 (e.g., 1 to 1000, 1 to 500, or 1 to 100) nucleotides, or a combination thereof;
(8) a bacteriophage having lytic activity against *Mycobacterium avium* and/or *Mycobacterium intracellulare,* wherein a genome of the bacteriophage comprises a nucleic acid sequence modified from a nucleic acid sequence of a genome of the bacteriophage identified by Accession No. NITE BP-03520 by deletion, substitution, insertion, or addition of 1 to 5000 (e.g., 1 to 1000, 1 to 500, or 1 to 100) nucleotides, or a combination thereo; and
(9) a bacteriophage having lytic activity against *Mycobacterium avium* and/or *Mycobacterium intracellulare,* wherein a genome of the bacteriophage comprises a nucleic acid sequence modified from a nucleic acid sequence of a genome of the bacteriophage identified by Accession No. NITE BP-03521 by deletion, substitution, insertion, or addition of 1 to 5000 (e.g., 1 to 1000, 1 to 500, or 1 to 100) nucleotides, or a combination thereof.

For the modifications such as deletion, substitution, insertion, or addition of a nucleotide(s), a plurality of modifications may be continuous, or a plurality of modifications may exist at different positions.

During passage culture, production, and/or replication of the bacteriophage, bacteriophage mutants in which the nucleic acid sequences of the genomes contained in the bacteriophage are partially deleted, substituted, inserted, and/or added may occur. Such mutants may be included in the bacteriophage of the present invention, as long as they have lytic activity against *Mycobacterium avium and*/*or Mycobacterium intracellulare.*

The bacteriophage of the present invention also includes a passage strain of a bacteriophage identified by any of above-described Accession Nos. NITE BP-03513 to NITE BP-03521, as long as it has lytic activity against *Mycobacterium avium* and/or *Mycobacterium*

### intracellulare.

As used herein, the term "passage strain" means a bacteriophage obtained by passage culture of a distributed bacteriophage.

The present invention may also include a polynucleotide consisting of the genome contained in the bacteriophage of the present invention (hereinafter referred to as "genome of the present invention"). Accordingly, the present invention provides: a polynucleotide of a bacteriophage genome that comprises a nucleic acid sequence having 90% or more identity to the nucleic acid sequence of the genome of a bacteriophage identified by any of Accession Nos. NITE BP-03513 to NITE BP-03517 and lacks an integrase gene and/or an immunity repressor gene; and a polynucleotide of a bacteriophage genome that comprises a nucleic acid sequence having 90% or more identity to the nucleic acid sequence of the genome of a bacteriophage identified by any of Accession Nos. NITE BP-03518 to NITE BP-03521. **In** one embodiment, the polynucleotide may be a polynucleotide encoding a bacteriophage having lytic activity against *Mycobacterium avium* and/or *Mycobacterium intracellulare.*

**In** one embodiment, the genome of the present invention may include a genome that: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03513; and (b) lacks an integrase gene and/or an immunity repressor gene.

**In** one embodiment, the genome of the present invention my include a genome that: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03514; and (b) lacks an integrase gene and/or an immunity repressor gene.

In one embodiment, the genome of the present invention may include a genome that: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03515; and (b) lacks an integrase gene and/or an immunity repressor gene.

In one embodiment, the genome of the present invention may include a genome that: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03516; and (b) lacks an integrase gene and/or an immunity repressor gene.

In one embodiment, the genome of the present invention may include a genome that: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03517; and (b) lacks an integrase gene and/or an immunity repressor gene.

In one embodiment, the genome of the present invention may include a genome that comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03518.

In one embodiment, the genome of the present invention may include a genome that comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03519.

In one embodiment, the genome of the present invention may include a genome that comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03520.

In one embodiment, the genome of the present invention may include a genome that comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03521.

The genome of the present invention may include a genome selected from (1) to (9) below:
(1) a genome that comprises a nucleic acid sequence of a genome of the bacteriophage identified by Accession No. NITE BP-03513;
(2) a genome that comprises a nucleic acid sequence of a genome of the bacteriophage identified by Accession No. NITE BP-03514;
(3) a genome that comprises a nucleic acid sequence of a genome of the bacteriophage identified by Accession No. NITE BP-03515;
(4) a genome that comprises a nucleic acid sequence of a genome of the bacteriophage identified by Accession No. NITE BP-03516;
(5) a genome that comprises a nucleic acid sequence of a genome of the bacteriophage identified by Accession No. NITE BP-03517;
(6) a genome that comprises a nucleic acid sequence of a genome of the bacteriophage identified by Accession No. NITE BP-03518;
(7) a genome that comprises a nucleic acid sequence of a genome of the bacteriophage identified by Accession No. NITE BP-03519;
(8) a genome that comprises a nucleic acid sequence of a genome of the bacteriophage identified by Accession No. NITE BP-03520; and
(9) a genome that comprises a nucleic acid sequence of a genome of the bacteriophage identified by Accession No. NITE BP-03521.

The genome of the present invention may include a genome selected from (1) to (9) below:
(1) a genome consisting of a nucleic acid sequence of a genome of the bacteriophage identified by Accession No. NITE BP-03513;
(2) a genome consisting of a nucleic acid sequence of a genome of the bacteriophage identified by Accession No. NITE BP-03514;
(3) a genome consisting of a nucleic acid sequence of a genome of the bacteriophage identified by Accession No. NITE BP-03515;
(4) a genome consisting of a nucleic acid sequence of a genome of the bacteriophage identified by Accession No. NITE BP-03516;
(5) a genome consisting of a nucleic acid sequence of a genome of the bacteriophage identified by Accession No. NITE BP-03517;
(6) a genome consisting of a nucleic acid sequence of a genome of the bacteriophage identified by Accession No. NITE BP-03518;
(7) a genome consisting of a nucleic acid sequence of a genome of the bacteriophage identified by Accession No. NITE BP-03519;
(8) a genome consisting of a nucleic acid sequence of a genome of the bacteriophage identified by Accession No. NITE BP-03520; and
(9) a genome consisting of a nucleic acid sequence of a genome of the bacteriophage identified by Accession No. NITE BP-03521.

The genome of the present invention can be produced using conventional techniques known in the art, such as recombinant DNA technology (e.g., polymerase chain reaction (PCR) amplification, cloning), enzymatic or chemical synthesis, or a combination thereof. For example, the genome of the present invention can be produced by ligating a plurality of polynucleotides containing the partial nucleotide sequences of the genome of the present invention by genetic engineering techniques. In one embodiment, the full-length of or partial sequence of the genome of the present invention may be contained in a vector known in the art.

The bacteriophage of the present invention can be obtained by making a request for distribution to the depository center.

Further, the bacteriophage of the present invention can be produced based on the sequence information by sequencing the nucleic acid sequence of the genome of the distributed bacteriophage by general techniques known in the art. For example, the genome of the present invention produced by the above method may be introduced into a host bacterium by electroporation (e.g., in the case of obtaining a bacteriophage having lytic activity against the genus *Mycobacterium,* the genus *Mycobacterium* may be used as the host bacterium). Then, the bacterium into which the genome has been introduced may be added to a plate overlaid with soft agar and cultured. Thereafter, single lytic plaques may be obtained by plaque assay. The bacteriophage of the present invention can be produced by adding the single lytic plaques to the host bacterial culture solution, followed by culturing and filtering the culture supernatant obtained by standing or centrifugation. Further, in order to produce the bacteriophage of the present invention, a lysogenic gene can be deleted from the phage genome using Bacteriophage Recombineering of Electroporated DNA (BRED) (PLoS One. 2008; 3 (12): e3957.).

The bacteriophage of the present invention can be prepared by conventional culture, isolation, and purification methods known in the art. For example, host bacteria (such as *Mycobacterium smegmatis, Mycobacterium avium,* or *Mycobacterium intracellulare*) may be pre-cultured, infected with the bacteriophage of the present invention, and cultured at 37°C. After culturing, the culture supernatant obtained by standing or centrifugation can be filtered, to obtain a purified bacteriophage. The medium can be appropriately selected depending on the bacterium to be used. For example, in the case of culturing *Mycobacterium smegmatis,* 7H9 medium can be used. In the case of preparing a plurality of bacteriophages, they may be grown in separate host bacteria, or they may be grown in the same host bacterium.

Further, the bacteriophage of the present invention can be stored in various forms (such as in liquid and freeze-dried products) by an appropriate method known in the art.

A person skilled in the art can also prepare a fusion of the bacteriophage of the present invention with a peptide, protein or modifier based on the present invention, and such bacteriophage fusion may also be included in the bacteriophage of the present invention. The peptide, protein and modifier used in the fusion may not be limited as long as the bacteriophage fusion has lytic activity against *Mycobacterium avium* and/or *Mycobacterium intracellulare,* and may include cell membrane-permeable peptides, tag peptides, toxins, antibodies or antigen-binding fragments thereof, liposomes, lipids, lipid nanoparticles, polyethylene glycols, sugar chains and low molecular weight compounds. The peptide, protein and modifier can be fused directly or indirectly to the bacteriophage of the present invention. When fusing indirectly, a linker or tag peptide can be used, for example.

The bacteriophage fusion can be readily produced by a person skilled in the art using methods known in the art, based on sequence information of the bacteriophage of the invention and information of a peptide, protein or modifier used in the fusion. For example, by infecting a host bacterium transformed with a plasmid carrying a exogenous gene encoding a protein of interest with the bacteriophage, a bacteriophage particle to which the protein of interest is fused can be produced.

### < 2. Uses of pharmaceutical composition and bacteriophage of present invention >

The bacteriophage of the present invention has lytic activity against non-tuberculous mycobacteria (NTM), particularly *Mycobacterium avium* and/or *Mycobacterium intracellulare,* which cause pulmonary MAC disease. Therefore, the bacteriophage of the present invention is expected to be effective for treating or preventing nontuberculous mycobacterial disease (NTM disease), particularly pulmonary MAC disease.

In another aspect, the present invention provides uses of the bacteriophage of the present invention, such as a pharmaceutical composition comprising the bacteriophage of the present invention as an active component (which may be hereinafter referred to as "pharmaceutical composition of the present invention").

The pharmaceutical composition of the present invention may include a pharmaceutical composition comprising the bacteriophage of the present invention and a pharmaceutically acceptable excipient.

The pharmaceutical composition of the present invention can be prepared using an excipient conventionally used in the art, that is, a pharmaceutical excipient, a pharmaceutical carrier, and the like by a conventional method. Examples of dosage forms of such a pharmaceutical composition may include parenteral agents such as injections, infusions, powder inhalers, and nebulizers, and it can be administered by intravenous administration, transpulmonary administration, or the like. For formulation, excipients, carriers, additives, or the like can be used depending on these dosage forms within a pharmaceutically acceptable range. For example, the pharmaceutical composition of the present invention can be produced by mixing the bacteriophage of the present invention with a pharmaceutically acceptable excipient or suspending the bacteriophage of the present invention in a pharmaceutically acceptable excipient.

The pharmaceutical composition of the present invention may include a pharmaceutical composition comprising at least two bacteriophages of the present invention and a pharmaceutically acceptable excipient.

The pharmaceutical composition of the present invention may include a pharmaceutical composition comprising three bacteriophages of the present invention and a pharmaceutically acceptable excipient.

In one embodiment, the pharmaceutical composition of the present invention may include a pharmaceutical composition comprising the bacteriophages of the following (1) to (3) and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks a lysogenic gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks a lysogenic gene; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

In one embodiment, the pharmaceutical composition of the present invention may include (1) to (3) below:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks an integrase gene and/or an immunity repressor gene; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

In one embodiment, the pharmaceutical composition of the present invention may include (1) to (3) below:
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519

In one embodiment, the pharmaceutical composition of the present invention may include (1) to (3) below:
(1) a bacteriophage wherein a genome of the bacteriophage consists of a nucleic acid sequence of a genome of the bacteriophage identified by Accession No. NITE BP-03513;
(2) a bacteriophage wherein a genome of the bacteriophage consists of a nucleic acid sequence of a genome of the bacteriophage identified by Accession No. NITE BP-03514; and
(3) a bacteriophage wherein a genome of the bacteriophage consists of a nucleic acid sequence of a genome of the bacteriophage identified by Accession No. NITE BP-03519.

In one embodiment, the pharmaceutical composition of the present invention may include (1) to (3) below:
(1) the bacteriophage identified by Accession No. NITE BP-03513;
(2) the bacteriophage identified by Accession No. NITE BP-03514; and
(3) the bacteriophage identified by Accession No. NITE BP-03519.

In one embodiment, the pharmaceutical composition of the present invention may include (1) to (3) below:
(1) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03513 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03514 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene; and
(3) a bacteriophage having lytic activity against *Mycobacterium avium* and/or *Mycobacterium intracellulare,* wherein a genome of the bacteriophage comprises a nucleic acid sequence modified from a nucleic acid sequence of a genome of the bacteriophage identified by Accession No. NITE BP-03519 by deletion, substitution, insertion, or addition of 1 to 5000 (e.g., 1 to 1000, 1 to 500, or 1 to 100) nucleotides, or a combination thereof.

For the modifications such as deletion, substitution, insertion, or addition of a nucleotide(s), a plurality of modifications may be continuous, or a plurality of modifications may exist at different positions.

The pharmaceutical composition of the present invention may include a passage strain of a bacteriophage identified by any of the above-described Accession No. NITE BP-03513, Accession No. NITE BP-03514, or Accession No. NITE BP-03519, as long as it has lytic activity against *Mycobacterium avium* and/or *Mycobacterium intracellulare.*

The effective dose, the administration frequency, and the administration period may vary depending on the purpose of administration (therapeutic or preventive purpose), the severity and age of the subject to be administered, the dosage form of the preparation to be used, the potency of the bacteriophage, and the like. For example, about 10⁴ to 10¹⁴ plaque forming unit (pfu) can be used as an effective dose of a single bacteriophage or as a total effective dose of two or more bacteriophages. The dosage ratio of the two or more bacteriophages can be appropriately adjusted according to the severity of symptoms and age of the patient, the dosage form of the preparation to be used, the potency of the bacteriophage, or the like. For example, in the case where the pharmaceutical composition comprises three bacteriophages, each bacteriophage may be contained in approximately equal amounts (e.g., 3.3 × 10³ to 3.3 × 10¹³ pfu), or each bacteriophage may be contained in different ratio.

The pharmaceutical composition of the present invention can be used as an agent for preventing or treating NTM disease, e.g., MAC disease or pulmonary MAC disease.

As used herein, "treat," "treating" and "treatment" mean at least partial improvement of symptoms of NTM disease (e.g., coughing, sputum, bloody sputum, fever, dyspnea, malaise, pulmonary nodules, and bronchiectasis), cessation of progress or deterioration of NTM disease including negative sputum culture, complete cure, and the like. As used herein, "prevent," "preventing" and "prevention" mean preventing a subject not suffering from NTM disease from developing NTM disease, preventing recurrence of NTM disease, and the like.

The present invention may include a pharmaceutical composition for preventing or treating NTM disease, e.g., MAC disease or pulmonary MAC disease, comprising the bacteriophage of the present invention or the pharmaceutical composition of the present invention.

Further, the present invention may include a method for preventing or treating NTM disease, e.g., MAC disease or pulmonary MAC disease in a subject, the method comprising a step of administering a therapeutically effective amount of the bacteriophage of the present invention or the pharmaceutical composition of the present invention. In one embodiment, at least two, e.g., three bacteriophages of the present invention can be administered to the subject, and the plurality of bacteriophages may be administered simultaneously or separately.

Further, the present invention may include the bacteriophage of the present invention or the pharmaceutical composition of the present invention for use in preventing or treating NTM disease, e.g., MAC disease or pulmonary MAC disease. Further, the present invention includes use of the bacteriophage of the present invention or the pharmaceutical composition of the present invention in the manufacture of a pharmaceutical composition for preventing or treating NTM disease, e.g., MAC disease or pulmonary MAC disease.

According to the present invention, the subject to which the bacteriophage is administered may not be limited, as long as it is a mammal, and examples thereof may include mice, rats, dogs, pigs, monkeys, and humans. For example, it may be administered to a subject diagnosed with NTM disease or a subject at risk of suffering from NTM disease.

The bacteriophage of the present invention or the pharmaceutical composition of the present invention may also be used or administered in combination with other components effective for treating or preventing NTM disease, and the bacteriophage of the present invention or the pharmaceutical composition of the present invention may be provided as a combination therapy with such other active components.

The present invention has been generally described above, but specific examples are now provided for reference for further understanding. These examples are for illustrative purposes and are not intended to limit the invention.

### [Examples]

Unless otherwise specified, the experiment was conducted according to the attached protocol for the parts using commercially available kits or reagents. Moreover, concentration mol/L is represented as M for convenience of description. For example, a 1M sodium hydroxide aqueous solution means a 1 mol/L sodium hydroxide aqueous solution.

### < Example 1: Preparation of bacteriophages >

Bacteriophages were isolated from natural environment samples by the following method. The natural environment sample (soil in Japan) was suspended in 10 mM Tris-HCl (pH 7.5) containing 1 mM CaCl₂, 10 mM MgSO₄, and 68.4 mM NaCl. The suspension was centrifuged, and the supernatant was collected. The supernatant was filtered with a 0.22 µm filter. The filtrate was mixed with *Mycobacterium smegmatis* and cultured with shaking using 7H9 medium for several hours to overnight. The culture solution was centrifuged, and the supernatant was collected. The supernatant was filtered with a 0.22 µm filter. The filtrate was mixed with *Mycobacterium smegmatis* and 7H9 medium containing 0.6% soft agar, and the mixture was overlaid on a 7H10 plate. It was cultured at 37 °C for several days. Lytic plaques appeared on the medium were collected and used as parent phages. The parent phages obtained were referred to as #33 parent strain, #63 parent strain, #105 parent strain, and #123 parent strain.

Next, the genomes were extracted from phages D29, B1, and Y2 (respectively referred to also as D29 parent strain, B1 parent strain, and Y2 parent strain) distributed from Okayama University and phages #33 parent strain and #63 parent strain obtained by the above method, and the entire nucleotide sequences were determined. Annotations were performed to identify lysogenic genes. Phages lacking an integrase gene and an immunity repressor gene, which are lysogenic genes, were obtained by any of the following methods. Specifically, 1) the target gene-deficient phage was obtained by deleting the lysogenic genes from the parent phage obtained above by the method of Marinelli, et al. (PLoS One. 2008; 3 (12): e3957.), or 2) the target gene-deficient phage was obtained by constructing a target phage genome (having a nucleotide sequence from which the lysogenic genes have been removed) from a plurality of polynucleotides having partial nucleotide sequences of the phage genome by genetic engineering techniques and introducing the phage genome into *Mycobacterium smegmatis* by electroporation. The bacteriophages obtained were respectively referred to as D29 lysogenic gene-deficient strain, B1 lysogenic gene-deficient strain, Y2 lysogenic gene-deficient strain, #33 lysogenic gene-deficient strain, and #63 lysogenic gene-deficient strain.

The genomes of the bacteriophages obtained above were extracted, and the entire nucleotide sequences were determined. The fact that the integrase gene and the immunity repressor gene were deleted was confirmed by comparison with the nucleotide sequence of the parent phage.

The accession numbers of the D29 lysogenic gene-deficient strain, B1 lysogenic gene-deficient strain, Y2 lysogenic gene-deficient strain, #33 lysogenic gene-deficient strain, #63 lysogenic gene-deficient strain, #105 parent strain, #123 parent strain, #33 parent strain, and #63 parent strain obtained above are respectively Accession Nos. NITE BP-03513, NITE BP-03514, NITE BP-03515, NITE BP-03516, NITE BP-03517, NITE BP-03518, NITE BP-03519, NITE BP-03520, and NITE BP-03521.

### < Example 2: Lytic activity of bacteriophage >

As a target bacterium, clarithromycin-resistant *Mycobacterium avium* clinical isolate KCH-ASGF-MAC-53 or KCH-ASGF-MAC-376 (distributed from Kinki Central Respiratory Center), or clarithromycin-resistant *Mycobacterium intracellulare* clinical isolate KCH-ASGF-MAC-27 or KCH-ASGF-MAC-90 (distributed from Kinki Central Respiratory Center) was grown on a 7H10 plate (19 g/L Middlebrook 7H10 Agar (Difco), 6.3 g/L glycerol, and 100 mL/L Middlebrook OADC Enrichment (Difco)). Bacterial colonies were scraped with a cotton swab and suspended in 7H9 medium (4.7 g/L Middlebrook 7H9 Broth (Difco)). Using a spectrophotometer, the bacterial suspension was adjusted to OD600 of about 1.5 to 2.0 with 7H9 medium. 700 µL of the bacterial suspension was mixed with 7 mL of 7H9 medium containing 0.6% soft agar (4.7 g/L Middlebrook 7H9 Broth (Difco), 6 g/L agarose, and 100 mL/L Middlebrook ADC Enrichment (Difco), or 4.7 g/L Middlebrook 7H9 Broth (Difco), 6 g/L agarose, and 100 mL/L Middlebrook OADC Enrichment (Difco)), and the mixture was overlaid on a 7H10 plate (length 100 mm × width 140 mm × height 14.5 mm).

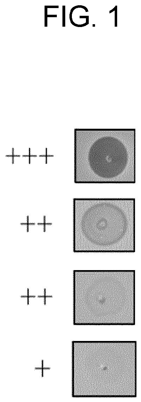

After confirming that the soft agar has completely gelatinized, D29 parent strain, D29 lysogenic gene-deficient strain, B1 parent strain, B1 lysogenic gene-deficient strain, Y2 parent strain, Y2 lysogenic gene-deficient strain, #33 parent strain, #33 lysogenic gene-deficient strain, #63 parent strain, #63 lysogenic gene-deficient strain, #105 parent strain, and #123 parent strain described in Example 1 were diluted with a phage buffer (10 mM Tris-HCl (pH 7.5) containing 1 mM CaCl₂, 10 mM MgSO₄, and 68.4 mM NaCl) to 10⁹ pfu/mL, and 2.5 µL was added dropwise. It was cultured at 37°C for about 3 to 7 days. The presence or absence of lytic plaques was confirmed by visual inspection. When lytic plaques were confirmed, transparent lytic plaques with little or no turbidity and clear contours were determined as +++, lytic plaques with clear contours and turbidity were determined as ++, and lytic plaques with unclear contours and turbidity were determined as + (see FIG. 1). Preliminary studies have already confirmed that adding a phage buffer alone to gelatinized soft agar does not cause lytic plaques.

The results are shown in Table 1. It was found that bacteriophages D29 parent strain, D29 lysogenic gene-deficient strain, B1 parent strain, B1 lysogenic gene-deficient strain, Y2 parent strain, Y2 lysogenic gene-deficient strain, #33 parent strain, #33 lysogenic gene-deficient strain, #63 parent strain, #63 lysogenic gene-deficient strain, #105 parent strain, and #123 parent strain exhibited lytic plaques against *Mycobacterium avium* and *Mycobacterium intracellulare.*

**[Table 1]**

| | Clarithromycin-resistant *Mycobacterium avium* | | Clarithromycin-resistant *Mycobacterium intracellulare* | |
|---|---|---|---|---|
| | KCH-ASGF-MAC-53 | KCH-ASGF-MAC-376 | KCH-ASGF-MAC-27 | KCH-ASGF-MAC-90 |
| D29 parent strain | +++ | +++ | +++ | +++ |
| D29 lysogenic gene-deficient strain | +++ | +++ | +++ | +++ |
| B1 parent strain | ++ | +++ | +++ | ++ |
| B1 lysogenic gene-deficient strain | +++ | +++ | +++ | +++ |
| Y2 parent strain | + | + | ++ | +++ |
| Y2 lysogenic gene-deficient strain | ++ | ++ | +++ | +++ |
| #33 parent strain | +++ | +++ | +++ | +++ |
| #33 lysogenic gene-deficient strain | +++ | +++ | +++ | +++ |
| #63 parent strain | +++ | ++ | ++ | ++ |
| #63 lysogenic gene-deficient strain | +++ | ++ | ++ | ++ |
| #105 parent strain | +++ | +++ | +++ | +++ |
| #123 parent strain | + | ++ | ++ | ++ |

From the above, it was confirmed that D29 parent strain, D29 lysogenic gene-deficient strain, B1 parent strain, B1 lysogenic gene-deficient strain, Y2 parent strain, Y2 lysogenic gene-deficient strain, #33 parent strain, #33 lysogenic gene-deficient strain, #63 parent strain, #63 lysogenic gene-deficient strain, #105 parent strain, and #123 parent strain had lytic activity against *Mycobacterium avium* and *Mycobacterium intracellulare.*

### < Example 3: Lytic activity of individual bacteriophage or phage cocktail over time >

About three bacterial colonies of *Mycobacterium smegmatis* grown on a 7H10 plate were scraped, suspended in 5 mL of 7H9 medium containing 0.05% Tween80 (NACALAI TESQUE, INC.), and then cultured with shaking at 37°C until OD600 of the bacterial suspension reached 3 or more using a spectrophotometer. After culturing, the operation of resuspending a precipitate from which the supernatant was removed by centrifugation in 7H9 medium was repeated three times, to give a bacterial suspension. The bacterial suspension was mixed with 7H9 medium containing 0.6% soft agar, D29 lysogenic gene-deficient strain, B1 lysogenic gene-deficient strain, or #123 parent strain (three strains) described in Example 1 was each added and mixed by inversion, and the mixture was overlaid on a 7H10 plate. After the soft agar was gelatinized, it was cultured at 37°C for about one day. 10 mL of the phage buffer was added to the plate and allowed to stand at room temperature for about 4 hours. The plate supernatant was filtered with a 0.22 µm filter, to obtain a phage lysate of each of the three strains. The pfu of the phage lysate was determined and stored at 4°C.

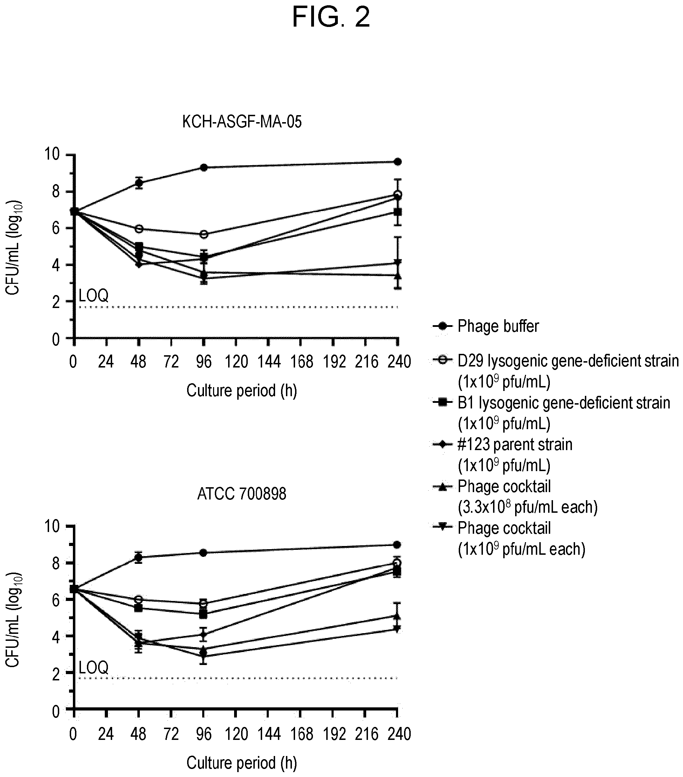

The phage lysate of each of the three strains was diluted with the phage buffer to 1 × 10¹⁰ pfu/mL or 3 × 10¹⁰ pfu/mL. In addition, as a phage cocktail of the three strains, they were mixed in equal amounts of each so that the total was 1 × 10¹⁰ pfu/mL, or the total was 3 × 10¹⁰ pfu/mL. 10 µL of these phage solutions or phage buffers were added to a 96 well plate per well. Two strains of *Mycobacterium avium* (KCH-ASGF-MA-05 (distributed from Kinki Central Respiratory Center) or ATCC 700898 (distributed from ATCC)) grown on a 7H10 plate at 37°C for 6 days were each scraped and suspended in 7H9 medium. The bacterial suspension was adjusted with 7H9 medium to a final OD600 of 0.0025 using a spectrophotometer, and 90 µL was added to a 96 well plate per well. A plate seal was attached to the 96 well plate as a lid and cultured at 37°C. Colony forming unit concentrations (CFU/mL) were calculated at 0, 48, 96, 240 hours after culturing. Three trials of phage evaluation were conducted. After converting the colony forming unit concentrations (CFU/mL) for each of the three trials into logarithms, the arithmetic mean ± standard error was calculated at each point (FIG. 2). CFU/mL after 240 hours was tested as one-way analysis of variance and Tukey's multiple comparisons (Table 2).

Table 2 shows the results of colony forming unit concentration (CFU/mL) test at 240 hours after culturing. The symbol * indicates that there was a significant difference among any two groups when tested at a significance level of less than 5% by Tukey's multiple comparison test (p < 0.05), the symbol ** indicates that there was a significant difference among any two groups by the above test (p < 0.01), the symbol *** indicates that there was a significant difference among any two groups by the above test (p < 0.001), and n.s. (not significant) indicates that there was no significant difference by the above test.

From the above, it was confirmed that, as a result of comparing the colony forming unit concentration (CFU/mL) at 0 hour and 96 hours after culturing, each phage and the phage cocktail of the three strains had the activity of reducing the number of bacteria against *Mycobacterium avium* at 0 hour after culturing (FIG. 2). Further, it was confirmed that this activity was maintained over a long period of time when the phage cocktail used, as compared with when each phage was used individually (FIG. 2 and Table 2).

**[Table 2]**

| **KCH-ASGF-MA-05** | Phage cocktail (1.0×10⁹ pfu/mL each) (total 3.0×10⁹ pfu/mL) | Phage cocktail (3.3×10⁸ pfu/mL each) (total 1.0×10⁹ pfu/mL) | #123 parent strain (1.0×10⁹ pfu/mL) | B1 lysogenic gene-deficient strain (1.0×10⁹ pfu/mL) | D29 lysogenic gene-deficient strain (1.0×10⁹ pfu/mL) |
|---|---|---|---|---|---|
| Phage buffer | ** | ** | n.s. | n.s. | n.s. |
| D29 lysogenic gene-deficient strain | * | * | n.s. | n.s. | |
| B1 lysogenic gene-deficient strain | n.s. | n.s. | n.s. | | |
| #123 parent strain | n.s. | * | | | |
| Phage cocktail (3.3×10⁸ pfu/mL each) (total 1.0×10⁹ pfu/mL) | n.s. | | | | |

| **ATCC 700898** | Phage cocktail (1.0×10⁹ pfu/mL each) (total 3.0×10⁹ pfu/mL) | Phage cocktail (3.3×10⁸ pfu/mL each) (total 1.0×10⁹ pfu/mL) | #123 parent strain (1.0×10⁹ pfu/mL) | B1 lysogenic gene-deficient strain (1.0×10⁹ pfu/mL) | D29 lysogenic gene-deficient strain (1.0×10⁹ pfu/mL) |
|---|---|---|---|---|---|
| Phage buffer | *** | *** | n.s. | n.s. | n.s. |
| D29 lysogenic gene-deficient strain | *** | *** | n.s. | n.s. | |
| B1 lysogenic gene-deficient strain | *** | ** | n.s. | | |
| #123 parent strain | *** | ** | | | |
| Phage cocktail (3.3×10⁸ pfu/mL each) (total 1.0×10⁹ pfu/mL) | n.s. | | | | |

### < Example 4: Efficacy of phage cocktail in mouse infection model >

Bacterial colonies of *Mycobacterium smegmatis* grown on a 7H10 plate were scraped, suspended in LB (Luria-Bertani) medium, followed by expanded culture at 37°C. After culturing, D29 lysogenic gene-deficient strain, B1 lysogenic gene-deficient strain or #123 parent strain, each of which was cultured and obtained by the method described in Example 3, was added and cultured at 37°C for about 1 day. The supernatant after culturing was filtered through a 0.22 µm filter to make phage lysates for each of the three strains. The respective lysates were then purified by ultracentrifugation to obtain phage solutions.

Purification by ultracentrifugation: DNaseI and RNaseA were added to each phage lysate to a final concentration of 1 µg/mL each and allowed to stand for 30 min at room temperature. NaCl was added to a final concentration of 1 M and dissolved, and the mixture was allowed to stand on ice or in a refrigerator for 1 h. Polyethylene Glycol 8000 was added to a final concentration of 10% w/v and dissolved, and the mixture was allowed to stand on ice or in a refrigerator for 2 h. The supernatant was removed by centrifugation at 11,000 g for 10 min at 4°C, and phage buffer was added to suspend and collect the precipitate. To the collected solution, 0.5 g of cesium chloride per mL was added, gently dissolved and transferred to an ultracentrifuge vessel. Cesium chloride solutions in a concentration of 1.45 g/mL, 1.5 g/mL and 1.7 g/mL each were injected sequentially into the bottom of the vessel using a syringe. The solution was centrifuged under the condition of 22,000 g for 2 h at 4°C, and the band containing the phage was collected. The collected phage-containing solution was replaced with phage buffer by dialysis, thereby preparing a phage solution. The pfu of the phage solution was determined and stored at 4°C.

The respective phage solutions of D29 lysogenic gene-deficient strain, B1 lysogenic gene-deficient strain and #123 parent strain, which were cultured and obtained by the method described above, were adjusted to 5 × 10¹¹ pfu/mL on the day of administration to mice. After adjustment, each phage solution was mixed at a ratio of 1:1:1 to prepare a solution for administering the phage cocktail.

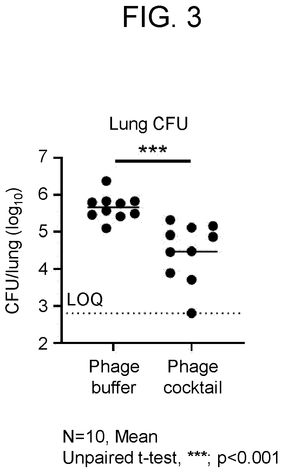

Clarithromycin-resistant *Mycobacterium intracellulare* KCH-ASGF-MAC-475 strain (distributed from Kinki Central Respiratory Centre), grown on 7H10 plates at 37°C for approximately 4 days, was scraped and suspended in saline. After suspension, the bacterial suspension was adjusted with saline to a final OD600 = 0.005 using a spectrophotometer. Twenty nude mice (Jackson Laboratory Japan, BALB/c-nu (nu/nu), 7 weeks old, female) were given 40 µL of bacterial suspension per mouse by intranasal administration and infected with the bacteria in the lungs. At 6-10 days, 13-17 days, 20-23 days and 27-31 days after infection, the phage buffer or the solution for administering phage cocktail was intranasally administered once per day (19 times in total) in a dose of 20 µL per mouse. 34 days after infection, mice were euthanased and lung tissue was collected. The lung tissue was dissociated in saline using gentleMACS^{™} Octo Dissociator (Miltenyi Biotech) to obtain dissociated solutions. The colony forming unit concentration (CFU/mL) of the dissociated solution was calculated and presented as the number of bacteria in the lung (CFU/lung) (FIG. 3).

FIG. 3 shows the results of the test of the number of bacteria in the lung (CFU/lung). The results show that when the difference between two groups was tested at a significance level of less than 5% using unpaired t-test, the phage cocktail group was significantly different from the phage buffer group (p < 0.001).

The results confirm that the bacteriophage cocktail has bactericidal activity in the in vivo lung infection model using mice.

### Industrial Applicability

The bacteriophage and the pharmaceutical composition of the present invention are expected to be useful for preventing or treating nontuberculous mycobacterial disease, e.g., MAC disease or pulmonary MAC disease.

### [Accession Nos.]

Accession No. NITE BP-03513 (bacteriophage D29 lysogenic gene-deficient strain D29Δ, deposited on August 26, 2021)
Accession No. NITE BP-03514 (bacteriophage B1 lysogenic gene-deficient strain B1Δ, deposited on August 26, 2021)
Accession No. NITE BP-03515 (bacteriophage Y2 lysogenic gene-deficient strain Y2Δ, deposited on August 26, 2021)
Accession No. NITE BP-03516 (bacteriophage #33 lysogenic gene-deficient strain #33Δ, deposited on August 26, 2021)
Accession No. NITE BP-03517 (bacteriophage #63 lysogenic gene-deficient strain #63Δ, deposited on August 26, 2021)
Accession No. NITE BP-03518 (bacteriophage #105 parent strain, deposited on August 26, 2021)
Accession No. NITE BP-03519 (bacteriophage #123 parent strain, deposited on August 26, 2021)
Accession No. NITE BP-03520 (bacteriophage #33 parent strain, deposited on August 26, 2021)
Accession No. NITE BP-03521 (bacteriophage #63 parent strain, deposited on August 26, 2021)

## Claims

1. A bacteriophage selected from (1) to (7) below:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks an integrase gene and/or an immunity repressor gene;
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03515; and (b) lacks an integrase gene and/or an immunity repressor gene;
(4) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03516; and (b) lacks an integrase gene and/or an immunity repressor gene;
(5) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03517; and (b) lacks an integrase gene and/or an immunity repressor gene;
(6) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03518; and
(7) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

2. The bacteriophage according to claim 1, which is selected from (1) to (7) below:
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514;
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03515;
(4) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03516;
(5) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03517;
(6) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03518; and
(7) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519.

3. The bacteriophage according to claim 1 or claim 2, which is selected from (1) to (7) below:
(1) a bacteriophage wherein a genome of the bacteriophage consists of a nucleic acid sequence of a genome of the bacteriophage identified by Accession No. NITE BP-03513;
(2) a bacteriophage wherein a genome of the bacteriophage consists of a nucleic acid sequence of a genome of the bacteriophage identified by Accession No. NITE BP-03514;
(3) a bacteriophage wherein a genome of the bacteriophage consists of a nucleic acid sequence of a genome of the bacteriophage identified by Accession No. NITE BP-03515;
(4) a bacteriophage wherein a genome of the bacteriophage consists of a nucleic acid sequence of a genome of the bacteriophage identified by Accession No. NITE BP-03516;
(5) a bacteriophage wherein a genome of the bacteriophage consists of a nucleic acid sequence of a genome of the bacteriophage identified by Accession No. NITE BP-03517;
(6) a bacteriophage wherein a genome of the bacteriophage consists of a nucleic acid sequence of a genome of the bacteriophage identified by Accession No. NITE BP-03518; and
(7) a bacteriophage wherein a genome of the bacteriophage consists of a nucleic acid sequence of a genome of the bacteriophage identified by Accession No. NITE BP-03519.

4. A bacteriophage selected from (1) to (7) below:
(1) the bacteriophage identified by Accession No. NITE BP-03513 or a passage strain thereof;
(2) the bacteriophage identified by Accession No. NITE BP-03514 or a passage strain thereof;
(3) the bacteriophage identified by Accession No. NITE BP-03515 or a passage strain thereof;
(4) the bacteriophage identified by Accession No. NITE BP-03516 or a passage strain thereof;
(5) the bacteriophage identified by Accession No. NITE BP-03517 or a passage strain thereof;
(6) the bacteriophage identified by Accession No. NITE BP-03518 or a passage strain thereof; and
(7) the bacteriophage identified by Accession No. NITE BP-03519 or a passage strain thereof.

5. A pharmaceutical composition comprising the bacteriophage according to any one of claims 1 to 4 and a pharmaceutically acceptable excipient.

6. A pharmaceutical composition comprising at least two bacteriophages according to any one of claims 1 to 4 and a pharmaceutically acceptable excipient.

7. A pharmaceutical composition comprising three bacteriophages according to any one of claims 1 to 4 and a pharmaceutically acceptable excipient.

8. The pharmaceutical composition according to any one of claims 5 to 7, which is a pharmaceutical composition for preventing or treating nontuberculous mycobacterial disease.

9. The pharmaceutical composition according to claim 8, wherein the nontuberculous mycobacterial disease is pulmonary MAC disease.

10. A pharmaceutical composition comprising the bacteriophages of the following (1) to (3) and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513; and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514; and (b) lacks an integrase gene and/or an immunity repressor gene; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

11. The pharmaceutical composition according to claim 10, comprising the bacteriophages of the following (1) to (3):
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519.

12. The pharmaceutical composition according to claim 10 or claim 11, comprising the bacteriophages of the following (1) to (3):
(1) a bacteriophage wherein a genome of the bacteriophage consists of a nucleic acid sequence of a genome of the bacteriophage identified by Accession No. NITE BP-03513;
(2) a bacteriophage wherein a genome of the bacteriophage consists of a nucleic acid sequence of a genome of the bacteriophage identified by Accession No. NITE BP-03514; and
(3) a bacteriophage wherein a genome of the bacteriophage consists of a nucleic acid sequence of a genome of the bacteriophage identified by Accession No. NITE BP-03519.

13. A pharmaceutical composition comprising the bacteriophages of the following (1) to (3) and a pharmaceutically acceptable excipient:
(1) the bacteriophage identified by Accession No. NITE BP-03513 or a passage strain thereof;
(2) the bacteriophage identified by Accession No. NITE BP-03514 or a passage strain thereof; and
(3) the bacteriophage identified by Accession No. NITE BP-03519 or a passage strain thereof.

14. The pharmaceutical composition according to any one of claims 10 to 13, which is a pharmaceutical composition for preventing or treating nontuberculous mycobacterial disease.

15. The pharmaceutical composition according to claim 14, wherein the nontuberculous mycobacterial disease is pulmonary MAC disease.
